# EUROPEAN PATENT SPECIFICATION

(11) **EP 0 732 318 B1**
(45) Date of publication and mention of the grant of the patent: **19.11.2003**
(21) Application number: 95610010.1
(22) Date of filing: 15.03.1995
(51) Int. Cl.: C07C 41/09, C07C 43/13, C11D 1/74

(54) **A process of preparing oligomeric and/or polymeric polyol compounds and their use in the production of emulsifying agents and surfactants**
Verfahren zur Herstellung von Oligomeren und/oder Polymerenpolyolverbindungen und ihre Anwendung in der Herstellung von Emulgatoren und oberflächenaktiven Mitteln
Procédé pour la préparation de composés de polyols oligomériques et/ou polymériques et leur utilisation dans la production d'émulsifiants et de tensioactifs

(43) Date of publication of application: 18.09.1996
(73) Proprietor: NEXUS A/S, DK-7130 Juelsminde (DK)
(72) Inventor: Norn, Viggo C., 7140 Stouby (DK); Christiansen, Kim, 8700 Horsens (DK)
(74) Representative: Plougmann & Vingtoft A/S

(56) References cited:
- WO-A-92/05133
- WO-A-93/02124
- WO-A-93/25511
- DE-A- 4 117 033
- US-A- 2 711 402
- CHEMICAL ABSTRACTS, vol. 106, no. 20, 1987 Columbus, Ohio, US; abstract no. 157038y, & JP-A-61 238 749 (NIPPON OILS AND FATS CO LTD)
- DATABASE WPI Week 9333 Derwent Publications Ltd., London, GB; AN 93-261605 & JP-A-05 178 777 (MITSUI TOATSU CHEM INC)

## Description

### FIELD OF INVENTION

The present invention relates to the provision of oligomeric or polymeric polyols which are useful as starting materials in the production of surface-active compounds including emulsifying agents, which are useful in the food industry. Particularly, there is provided a novel method for preparing such polyols.

### TECHNICAL BACKGROUND AND PRIOR ART

Oligomers and/or polymers of polyols (or polyhydric alcohols) or mixtures of such oligomers and/or polymers are currently used as starting materials in the production of food emulsifying agents and surfactants. In this connection, widely used oligomers and/or polymers are prepared from the trihydric alcohol glycerol (propane-1,2,3-triol). In the industry, such starting materials prepared from glycerol are generally known as polyglycerols, irrespective of the degree of oligo- or polymerization.

Polyglycerols can be esterified with fatty acids through the free hydroxyl groups providing a broad range of edible emulsifying agents having differing hydrophilic/lipophilic balance (HLB)-values.

Polyglycerols which have been known for several decades are manufactured by an oligo- or polymerization of glycerol, a process which is also normally referred to as condensation. In this process, the polyol starting materials are reacted in the presence of a catalyst which is typically an alkaline substance, resulting in a mixture of non-oligomerized glycerol and different oligomers. Of such oligomers, di-, tri- tetra- and pentamers are particularly useful as starting materials for subsequent preparing of emulsifying agents. A review of the conventional preparation of polyglycerols as well as the subsequent esterification step is given by R.T. McIntyre in Journal of American Oil Chemist Society, 1979, **56,** 835A-840A.

Examples of alkaline substances which are currently used as catalysts in the above polyol condensation process are NaBO₂, KOH, NaSnO₄, NaOH, LiOH, Na₂SiO₂ and silica gel.

Thus, DE 40 29 323 discloses a glycerol condensation process using silicates as catalysts, resulting in a product with a high content of dimerized glycerol, and in DE 41 17 033 a process for preparing polyglycerols using alkaline borates, e.g. sodium metaborate, is described. WO 93/25511 discloses a process of producing a glycerol condensate product having a high diglycerol content in which the catalyst is selected from zeolites, especially alkaline types hereof.

In general, the prior art processes of preparing polyglycerols require long reaction times at high temperatures, which may typically be up to 280°C, in order to obtain polyglycerol mixtures predominantly consisting of the most desired oligomers. These known processes, although they may provide end products having a suitable composition with regard to glycerol oligomers (condensates), involve several disadvantages of which the most important are: (i) the long reaction time and/or the high process temperatures required renders the processes costly because of the high energy consumption and (2) these process conditions result in end products which have an undesired dark colour, which may limit their practical use in connection with food production.

It has now surprisingly been found that when sodium aluminate, NaAlO₂ is used as a catalyst in the oligo- and/or polymerization of polyols, the reaction temperature and time can, relative to currently used process conditions, be reduced significantly, and additionally, the use of sodium aluminate results in end products having a desired composition of oligomeric polyol components. Relative to the prior art, the present invention has demonstrated that polyol condensation reactions catalyzed by sodium aluminate result in substantially higher reaction rates, i.e. the time . required to obtain polyol oligomers and/or polymers having the desired degree of oligo- or polymerisation is substantially reduced. Furthermore, oligo- or polymerization reactions employing conventional catalysts generally require considerably longer reaction time at equivalent amounts to obtain comparable desired end product compositions than NaAlO₂ catalyzed reactions do.

Furthermore, and importantly, the products resulting from a process in which NaAlO₂ is used as a catalyst have highly desirable colour characteristics as compared to products provided by the prior art processes, i.e. the products are less coloured than known products. In particular, products according to the present invention have less content of red colour tones which are undesirable in oligo- or polymeric polyol products.

### SUMMARY OF THE INVENTION

Accordingly, the present invention relates in a first aspect to a process for preparing an oligomeric and/or polymeric polyol compound, said oligomeric and polymeric polyol compounds being linked by ether bonds, the method comprising reacting a polyol starting material with an amount of NaAlO₂ which causes the polyol to form at least dimeric oligomers with concomitant formation of H₂O, the method optionally comprising the further step of removing the NaAlO₂, e.g. by treating the reaction mixture with an ion exchange resin.

The process of the invention provides a composition comprising oligomers and/or polymers of glycerol which composition, when prepared by reacting glycerol or oligomers thereof with about 3.5% (w/w) of NaAlO₂ at a temperature of about 240 °C and a pressure of about 850 mBar for about 5 hours, has a colour number of at the most 10 when measured by a Lovibond tintometer as described herein.

A surface-active composition is obtainable by esterifying a composition as defined with a fatty acid.

### DETAILED DISCLOSURE OF THE INVENTION

The process according to the present invention provides a composition comprising oligomers and/or polymers of a polyol. In the present context, the term "oligomer" is used to describe a compound which consists of two and up to seven or eight polyol molecules which are linked by ether bondings, whereas the term "polymers" as used herein describes polyol compounds consisting of a higher number of monomeric units, also linked by ether bondings. In the art, such oligomeric or polymeric compounds are also referred to as polyol condensates.

In the above process, a polyol starting material is reacted with an amount of NaAlO₂ which is sufficient to cause the polyol to form at least dimeric oligomers with concomitant formation of H₂O. One preferred useful polyol starting material is glycerol, which may be of natural or synthetic origin, but is preferably a material which meet the B.P. quality standards or which is even of a higher quality. It is, however, contemplated that other polyols such as e.g. polyethylene glycols, sugar alcohols and fatty alcohols such as dihydroxy fatty alcohols can be used as starting materials in accordance with the invention. The starting material may also be a mixture of different polyol monomers or it can be oligomeric polyols or mixtures hereof.

In accordance with the invention, the polyol starting material is reacted under the below conditions with the appropriate amount of sodium aluminate in a suitable reaction vessel provided with stirring means and connected to trapping means for condensing the water which is released during the reaction. The vessel and the condensing means connected hereto is connected to a vacuum pump to provide pressures below 1 Bar.

The NaAlO₂ is typically used in an amount which is in the range of 0.01-20% (w/w), preferably in the range of 0.1-10% (w/w) and more preferably in the range of 0.3-3.5%, calculated on the amount of polyol starting material. Accordingly, in useful embodiments of the invention, the ratio between catalyst and the polyol starting material is in the range of 1:10000 to 1:5, such as in the range of 1:1000 to 1:10.

The reaction is suitably carried out at a temperature which is in the range of 150-300°C, such as in the range of 175-275°C and preferably in the range 230-250°C. The reaction proceeds normally at a pressure below 1 Bar which is provided by the above vacuum pump connected to the reaction vessel, such as e.g. in the range of 10-990 mBar and preferably in the range of 50-750 mBar such as in the range of 500-950 mBar.

The reaction time depends on the starting material, other reaction parameters and the desired end product, but is typically in the range of 1-50 hours, such as in the range of 2-25 hours e.g in the range of 5-15 hours.

It has found that the reaction may proceed at an enhanced rate if the water being released during the reaction is continuously removed and accordingly, the process comprises in a preferred embodiment the step of continuously removing the generated water from the reaction mixture by evaporating and condensing the water vapour. The amount of water being generated during the reaction can be measured in order to monitor the progress of the reaction.

Prior to and/or during the reaction, the reaction mixture may advantageously be sparged with an inert gas such as nitrogen.

Thus, the gas may e.g. be bubbled through the reaction mixture for 5-60 minutes at atmospheric pressure and ambient temperature, i.e. prior to heating and operating the vacuum pump. The sparging may also be continued during the reaction at elevated temperatures and reduced pressures.

The reaction is allowed to proceed until a desired degree of oligo- or polymerisation is achieved at which point of time the reaction is terminated by cooling of the reaction mixture to temperatures below 100°C. The cooling may be provided by any known method such as circulation of a cold liquid around the reaction vessel, or by air cooling.

It has significantly been found that the reaction rate can be increased by adding fresh polyol starting material once or at intervals during the reaction. Accordingly, the process according to the present invention provides in a further preferred embodiment a process which includes the addition of further polyol during the reaction. Such a process is described in details in the below examples. The amount of fresh polyol added per addition is typically in the range of 1:100 to 1:1 relative to the starting amount of polyol.

The process results in an end product which is a mixture of substances selected from monomers, dimers, trimers, tetramers and higher oligomers of the polyol being reacted, or even polymers of the polyol. As mentioned above, glycerol is a preferred polyol starting material and accordingly, the process is preferably one wherein the polyol being reacted is glycerol or a oligomer hereof.

NaAlO₂ is soluble in polyols such as in glycerol. Accordingly, it is normally required that following the reaction, the catalyst be separated from the reaction mixture. This removal may be carried out by any suitable separation method such as separation by contacting the reaction mixture with an ion exchange substance including a resin substance, or by adding an acid or any other compound to the reaction mixture to precipitate the catalyst, followed by separating the precipitate by any known method of separating solid materials from liquid media. Optionally, the reaction mixture is neutralized by adding an organic or inorganic acid such as phosphoric acid, hydrochloric acid or sulphuric acid.

Furthermore, for certain purposes it may be required to subject the resulting end product to a bleaching treatment. This can e.g. suitably be done by contacting the end product with an absorbing agent such as activated carbon.

Although the above end product comprising a mixture of monomeric and oligomeric polyol substances is immediately useful as starting materials in the production of emulsifying agents as mentioned above, it may for certain purposes be advantageous to provide such starting materials in the form of one or more isolated individual oligomers e.g. dimers, trimers or tetramers. Such a separation step can typically be carried out by distilling the end product resulting from the above process, preferably a distilling in vacuum such as short path distillation or thin layer distillation.

The end products resulting from the process according to the invention are, as it is also mentioned above, particularly useful as starting materials for preparing surface-active compounds, in particular emulsifying agents. Accordingly, the present invention also provides a process which comprises the further step of esterifying the oligomeric and/or polymeric polyol compounds resulting from the above process, or a fraction hereof with one or more fatty acids to obtain the surface-active emulsifying composition. In this connection, particularly suitable fatty acids include long chain fatty acids such as C₆₋₂₄ fatty acids, although preferred fatty acids are selected from C₁₆₋₁₈ fatty acids. The fatty acid may be saturated, mono- or polyunsaturated depending on the desired characteristics of the emulsifying agent to be obtained.

The present process also provides a surface-active composition obtainable by esterifying a composition as defined above with a fatty acid.

The present process provides a composition comprising oligomers and/or polymers of glycerol which composition, when prepared by reacting the polyol or oligomers thereof with about 3.5% (w/w) of NaAlO₂ at a temperature of about 240°C and a pressure of about 850 mBar for about 5 hours, has a colour number of at the most 10 when measured by a Lovibond tintometer as described herein. In this method, the red colour tone and the yellow colour tone, respectively are measured, and a combined, weighted colour number is calculated as described below.

Although it is highly desired to have such compositions which are substantially without red and/or yellow colour tones as measured according to the above method, i.e. having a colour tone in the range of 0-1, compositions having higher colour numbers may be acceptable. However, the colour number of such a composition is preferably at the most 9, such as at the most 8 or more preferably at the most 7, including at the most 6, e.g. at the most 6. It is even more preferred that the composition has a colour as defined above which is at the most 5, such as at the most 4. Particularly useful compositions according to the invention have a colour number which is even lower, including at the most 3, such as at the most 2 or at the most 1.

### EXAMPLE 1

### Preparation of compositions comprising oligomers and/or polymers of glycerol, using different amounts of NaAlO₂

Three reaction mixtures of glycerol and sodium aluminate containing 0.5, 1.5 and 3.0% (w/w), respectively of the catalyst (calculated on glycerol) were transferred to separate reaction flasks provided with a stirrer, a thermometer and a condenser connected to a vacuum pump. Nitrogen was bubbled through the reaction mixtures for about 5 minutes. Subsequently, the reaction mixtures were heated for about 10 hours under stirring conditions to about 240°C while the pressure was reduced to and kept at about 850 mBar using a mechanical vacuum pump. During the reaction, samples of the reaction mixtures were withdrawn every hour.

After 10 hours the reaction was stopped by cooling the reaction mixture to room temperature. The resulting end product is a very viscous yellow liquid comprising a mixture of oligomers of glycerol.

The samples were analyzed by gas liquid chromatography (GLC) and the results are summarized in the below Tables 1-3:

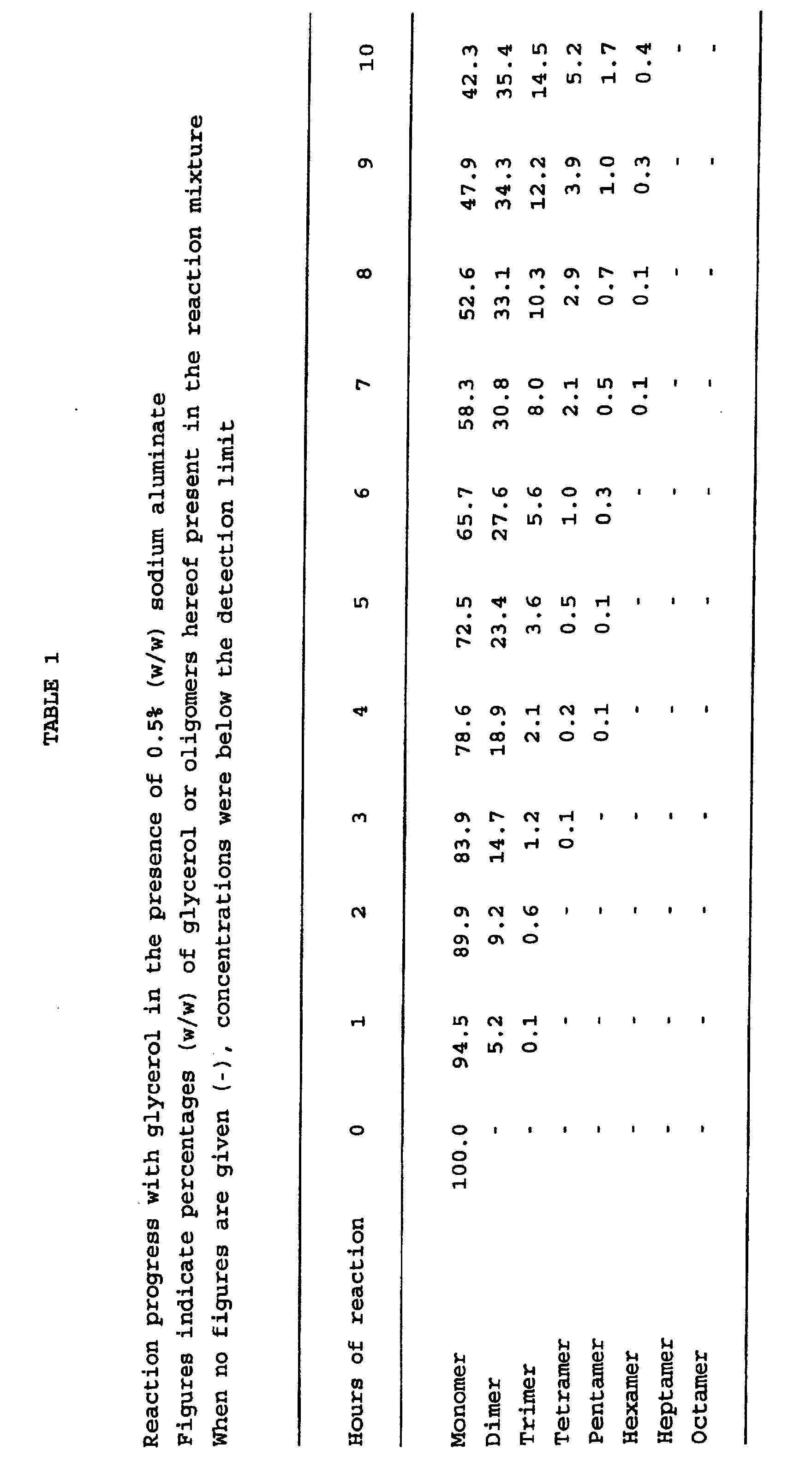

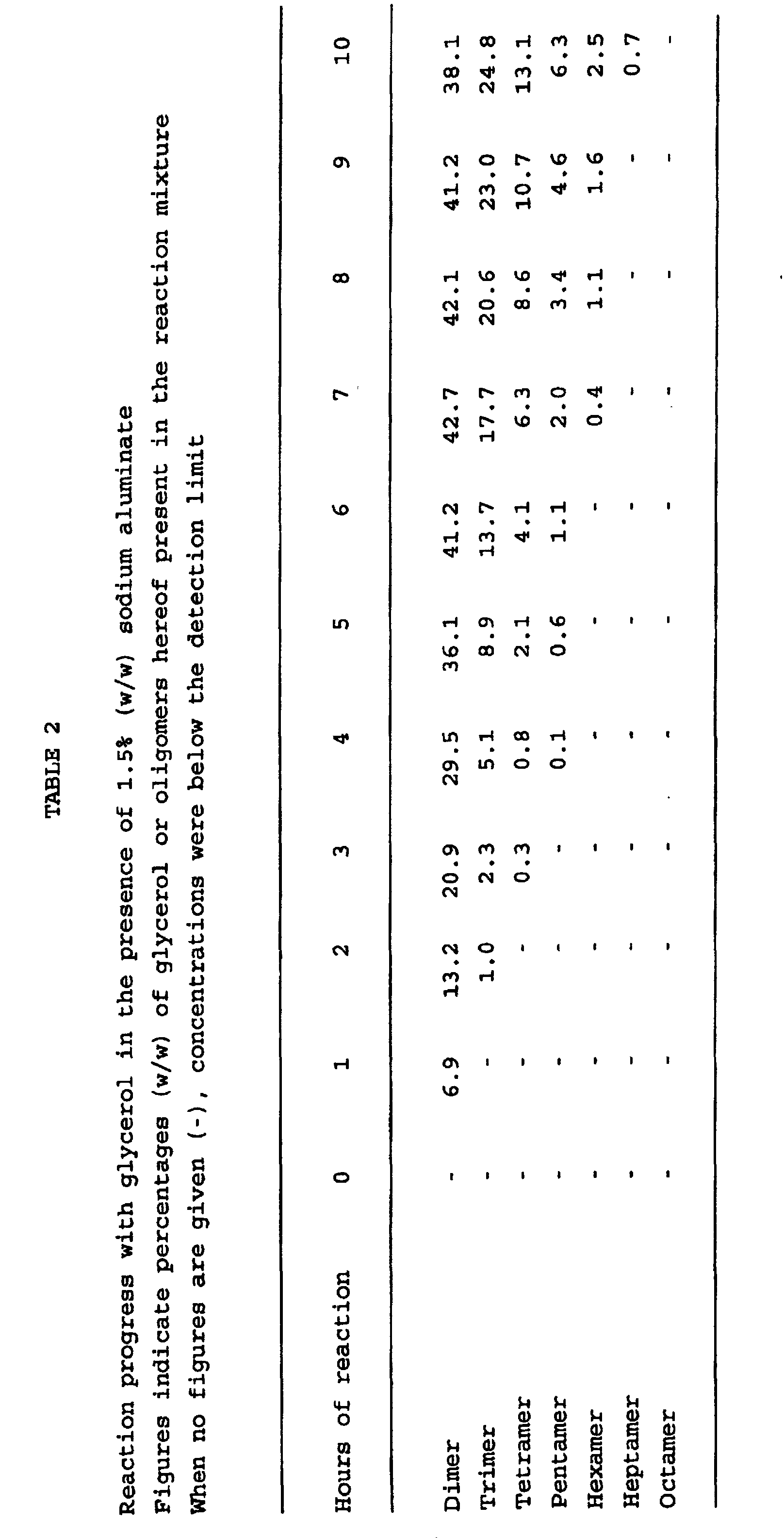

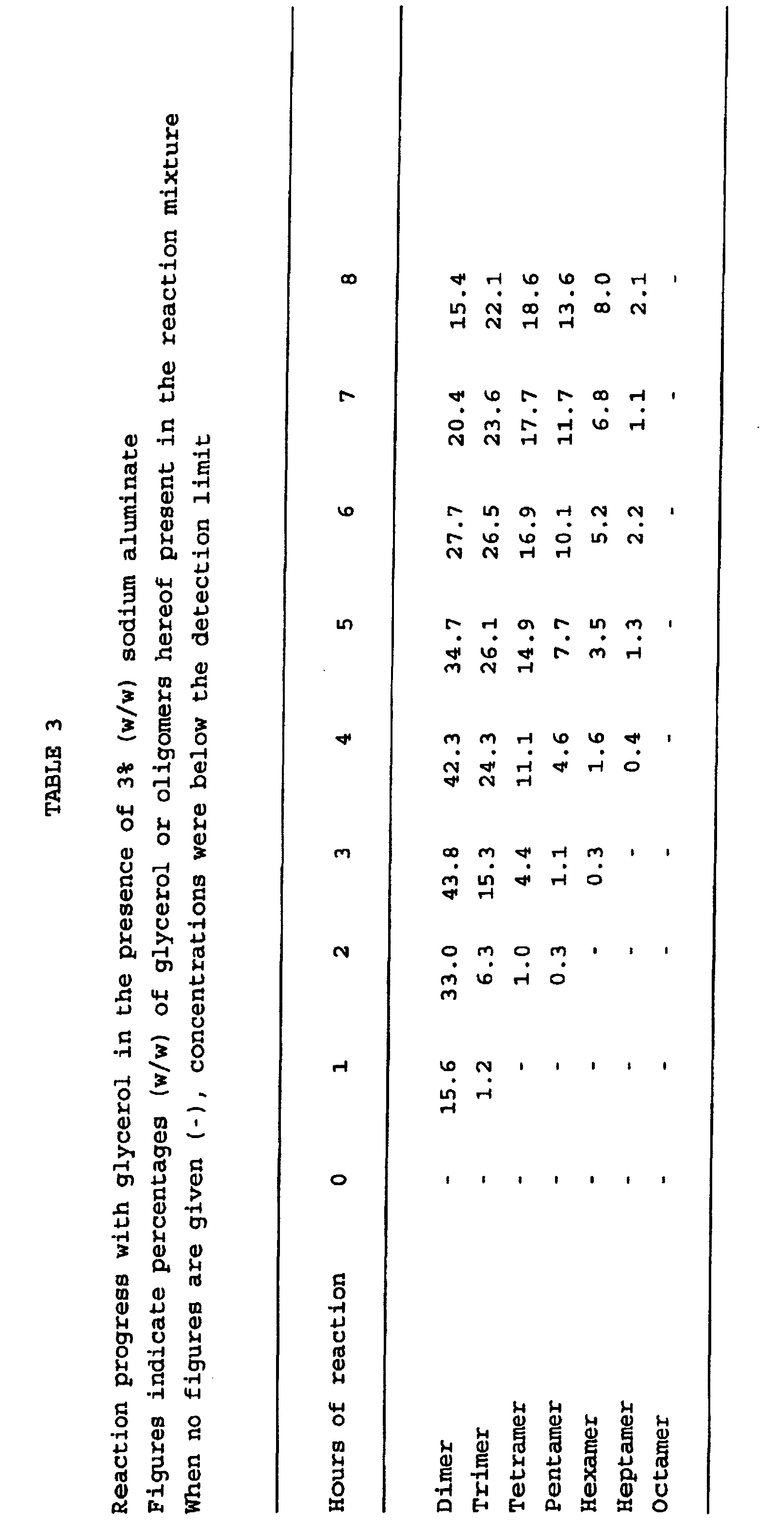

For comparison, a similar experiment using 1.5% (w/w) of NaOH as the catalyst in place of NaAlO₂ was carried out. The results hereof are summarized in Table 4 below:

| Reaction progress with glycerol in the presence of 1.5% (w/w) sodium hydroxide. Figures indicate percentages (w/w) of glycerol or oligomers hereof present in the reaction mixture. When no figures are given (-), concentrations were below detection limit. | | | | | | |
|---|---|---|---|---|---|---|
| Hours of reaction | 0 | 1 | 2 | 3 | 4 | 5 |
| Dimer | - | 0.2 | 4.3 | 13.8 | 22.8 | 28.4 |
| Trimer | - | - | - | 1.4 | 4.7 | 7.7 |
| Tetramer | - | - | - | - | 0,8 | 1.9 |
| Pentamer | - | - | - | - | 0.1 | 0.9 |
| Hexamer | - | - | - | - | - | - |
| Heptamer | - | - | - | - | - | - |
| Octamer | - | - | - | - | - | - |

When e.g. comparing the data in Table 2 and Table 4 it is apparent, that the reaction rate is significantly higher in the test reaction mixture containing 1.5% NaAlO₂ as compared to the control. After 5 hours the proportion of the desired dimers, trimers and tetramers was 47.1% using NaAlO₂ as compared to the corresponding value for NaOH which was 38.0%. Furthermore, the colour of the test reaction mixtures after 5 hours of reaction were visually considerably less dark than the reaction mixture containing NaOH.

### EXAMPLE 2

### Preparation of composition comprising oligomers and/or polymers of glycerol during which additional glycerol is added

73.6 kg of glycerol was pumped into a stainless steel reactor equipped with stirrer, condenser and connected to a vacuum pump. 2.3 kg sodium aluminate was added to the glycerol. The resulting reaction mixture was sparged with nitrogen and after 5 minutes the vacuum pump was started and heating provided by circulating hot oil around the reactor. The reaction was kept constant at about 245°C and at about 680 mBar for about 5 hours, followed by the addition of about 11 kg of monomeric glycerol. The reaction was continued for about another 5 hours and a further amount of about 11 kg of glycerol was added and the reaction continued for about 5 hours.

The reaction was then stopped by cooling the reaction mixture to about 90°C and 75.1 kg of an end product in the form of a viscous yellow liquid was obtained. Gas liquid chromatography (GLC)-analysis of the end product revealed the following composition hereof in percentages (w/w), calculated on the mixture:

| | |
|---|---|
| 21.1% | monomeric glycerol |
| 41.2% | dimeric glycerol |
| 21.8% | trimeric glycerol |
| 9.6% | tetrameric glycerol |
| 3.7% | pentameric glycerol |

This experiment illustrates that a process as used in this example results in a mixture of polyglycerol oligomers having a total content of dimers, trimers and tetramers which is higher than 70% (w/w) of the end product.

### EXAMPLE 3

### A comparison of the efficiency of different catalysts

In this experiment, the efficiency with regard to reaction rate of a number of catalysts currently used in the production of oligomeric glycerol compounds was compared with that of NaAlO₂. Furthermore, the end products obtained was subjected to a determination of colour number using a Lovibond tintometer. According to this method, the red colour tone and the yellow colour tone is measured separately by comparing the colour tone of a sample in a 1 inch cell with a colour tone scale. Based on these visual characterizations, a combined (weighted) colour number was calculated by dividing the yellow colour tone values by 10 and adding these values to the respective values for the red colour tone.

Two moles of glycerol (184.18 g) and 0.08 moles of catalyst (6.56 g in the case of NaAlO₂) were reacted for 5 hours essentially as described in Example 1. The compositions of the resulting end products are summarized in Table 5 below.

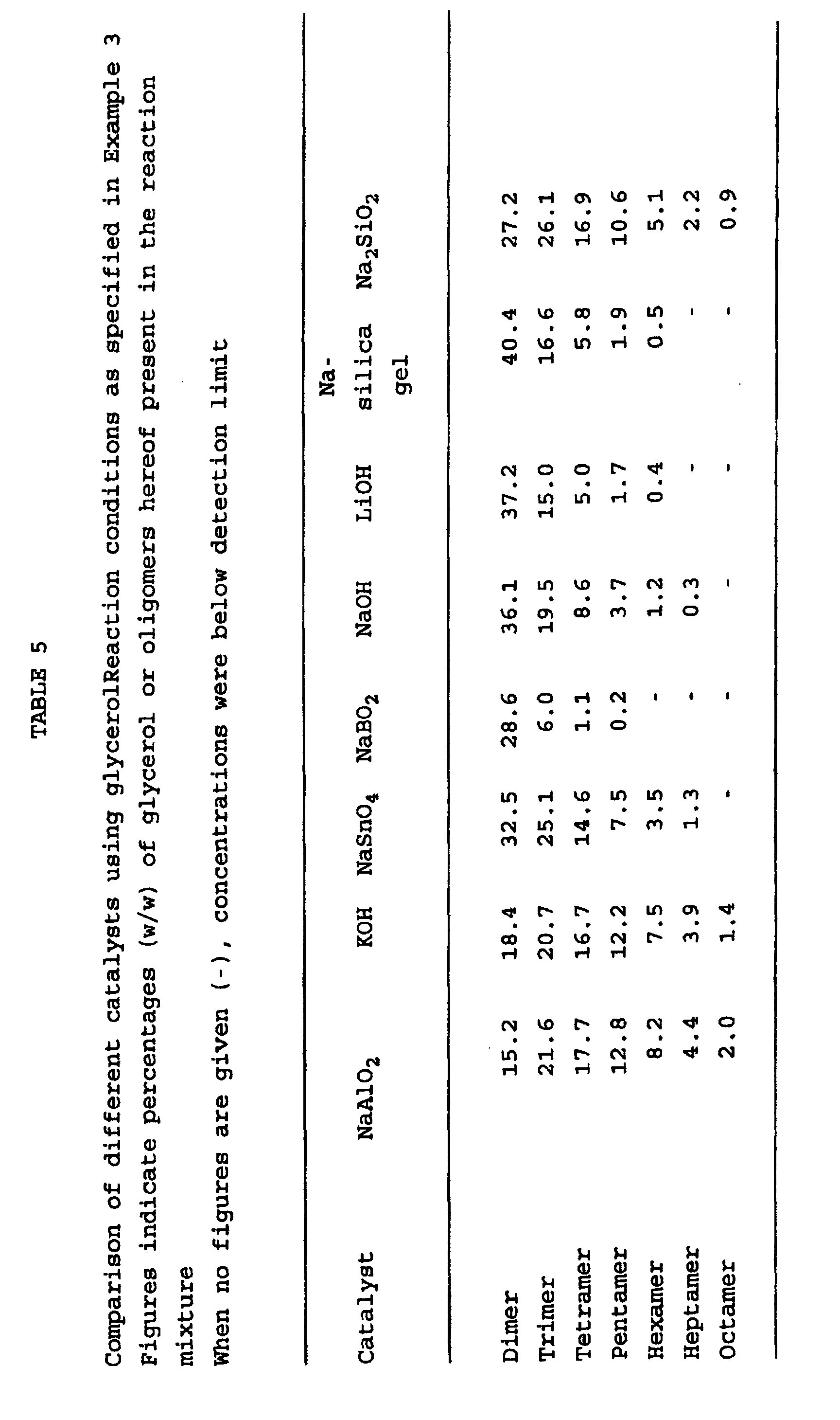

The above results clearly demonstrate the efficiency of sodium aluminate with regard to providing a high proportion of the desired oligomeric compounds at high reaction rates in comparison to the known catalysts, with the exception of KOH. However, it was found that of the tested catalysts, only NaAlO₂ resulted in an end product having a colour number less than 10 as it is illustrated in Table 6 below. Relative to KOH, the colour number obtained with NaAlO₂ was reduced by more than 2.0 which from a practical point of view is a significant improvement.

In order to demonstrate inherent differences between NaAlO₂ and KOH as catalysts of the reaction in question, further experimentation was performed as described in the below Example 4.

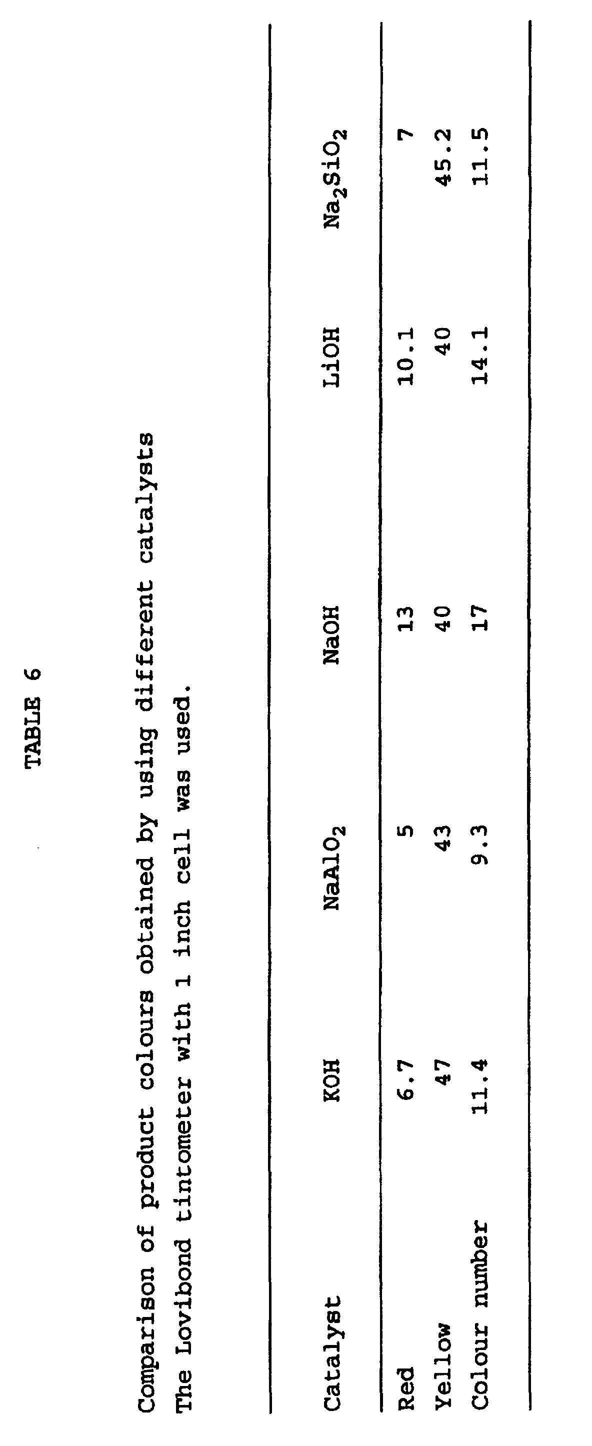

### EXAMPLE 4

As a composition of about 25% dimers and 25% trimers and somewhat less of tetra-, penta- and hexamers was particularly desired, an experiment illustrating the differences between NaAlO₂ and KOH as catalysts in achieving this composition was performed. Unless otherwise stated, reaction conditions were as specified in Example 1.

**TABLE 7**

| Reaction time needed to achieve desired composition Figures for glycerol oligomers denote percentages (w/w) of the listed oligomers | | |
|---|---|---|
| Catalyst | NaAlO₂ | KOH |
| Mole catalyst pr. mole glycerol | 0.035 | 0.04 |
| Reaction time | 6.5 hours | 10 hours |
| Dimer | 24 | 24 |
| Trimer | 25 | 23 |
| Tetramer | 17 | 16 |
| Pentamer | 11 | 10 |
| Hexamer | 6 | 5 |

When two glycerol molecules are being linked as in the present invention, three isomers may be formed. Primary alcohol functional groups of both molecules involved may be linked (alpha-alpha-isomer), primary and secondary alcohol functional groups may be linked (alpha-beta-isomer) or two secondary alcohol functional groups may be linked (beta-beta-isomer).

**TABLE 8**

| Composition of dimeric isomers of glycerol produced as specified in Example 3 and analyzed by GLC Figures denote percentages (w/w) of dimerized glycerol isomers | | | |
|---|---|---|---|
| | Alpha-alpha | Beta-alpha | Beta-beta |
| NaAlO₂ | 41.5 | 42.7 | 15.8 |
| KOH | 36.8 | 42.7 | 20.5 |

From this example, the superiority of NaAlO₂ over KOH with respect to reaction progression and selectivity was clearly demonstrated. When using essentially similar molar concentrations of the catalysts NaAlO₂ and KOH, respectively, extended reaction time was needed when using KOH in order to produce end products of comparable, desired compositions.

Alpha-alpha isomers are particularly favoured over corresponding isomers. Broader ranges of useful emulsifying characteristics of emulsifying agents manufactured from such isomers, e.g. HLB-values, crystal structure and crystallization-promoting properties are provided, when high proportions of alpha-alpha linkage isomers are present in the starting material. As is apparent from Table 8, NaAlO₂ can catalyze alpha-alpha bonding formation more than 10% more effectively than KOH.

## Claims

1. A process of preparing a composition comprising oligomers and/or polymers of a polyol, said oligomers and polymers being linked by ether bonds, the process comprising reacting a polyol starting material with an amount of NaAlO₂ which causes the polyol to form at least dimeric oligomers with concomitant formation of H₂O.

2. A process according to claim 1 wherein the H₂O being formed is continuously removed.

3. A process according to claim 1 wherein further polyol is added during the reaction.

4. A process according to any of preceding claims wherein NaAlO₂ is used in an amount which is in the range of 0.1-10% (w/w) calculated on the amount of polyol starting material.

5. A process according to claim 4 wherein NaAlO₂ is used in an amount which is in the range of 0.3-3.5%.

6. A process according to any of preceding claims wherein the temperature at which the polyol is reacted is in the range of 200°C to 270°C.

7. A process according to any of preceding claims wherein the reaction is carried out at a pressure below 1 Bar.

8. A process according to any of preceding claims wherein the resulting composition is a mixture of substances selected from monomers, dimers, trimers, tetramers and higher oligomers of the polyol.

9. A process according to any of preceding claims wherein the polyol being reacted is glycerol or an oligomer thereof.

10. A process according to claim 8 wherein the resulting composition is separated into monomers, dimers, trimers, tetramers or higher oligomers of the polyol.

11. A process according to claim 1 which further comprises the step of esterifying the resulting composition or a fraction thereof with a fatty acid to obtain a surface-active composition.

12. A process according to any of the preceding claims wherein the method comprising the further step of removing the NaAlO₂.

13. A process according to claim 11, wherein the removing of NaAlO₂ is conducted by treating the reaction mixture with an ion exchange resin

## Patentansprüche

1. Verfahren zur Herstellung einer Zusammensetzung, welche Oligomere und/oder Polymere eines Polyols umfasst, wobei die Oligomere und Polymere durch Etherbindungen verknüpft sind, wobei das Verfahren umfasst, ein Polyol-Ausgangsmaterial mit einer Menge von NaAlO₂, welche bewirkt, dass das Polyol unter gleichzeitiger Bildung von H₂O wenigstens dimere Oligomere bildet, umzusetzen.

2. Verfahren nach Anspruch 1, wobei das H₂O, das gebildet wird, kontinuierlich entfernt wird.

3. Verfahren nach Anspruch 1, wobei während der Umsetzung weiteres Polyol zugesetzt wird.

4. Verfahren nach einem der vorangegangenen Ansprüche, wobei NaAlO₂ in einer Menge verwendet wird, welche im Bereich von 0,1-10% (Gew./Gew.), berechnet ausgehend von der Menge des Polyol-Ausgangsmaterials, liegt.

5. Verfahren nach Anspruch 4, wobei NaAlO₂ in einer Menge verwendet wird, die im Bereich von 0,3-3,5% liegt.

6. Verfahren nach einem der vorangegangenen Ansprüche, wobei die Temperatur, bei welcher das Polyol umgesetzt wird, im Bereich von 200°C bis 270°C liegt.

7. Verfahren nach einem der vorangegangenen Ansprüche, wobei die Umsetzung bei einem Druck unter 1 bar ausgeführt wird.

8. Verfahren nach einem der vorangegangenen Ansprüche, wobei die resultierende Zusammensetzung eine Mischung von Substanzen, ausgewählt aus Monomeren, Dimeren, Trimeren, Tetrameren und höheren Oligomeren des Polyols, ist.

9. Verfahren nach einem der vorangegangenen Ansprüche, wobei das Polyol, welches umgesetzt wird, Glycerol oder ein oligomer davon ist.

10. Verfahren nach Anspruch 8, wobei die resultierende Zusammensetzung in Monomere, Dimere, Trimere, Tetramere oder höhere Oligomere des Polyols aufgetrennt wird.

11. Verfahren nach Anspruch 1, welches ferner den Schritt umfasst, die resultierende Zusammensetzung oder einen Teil von dieser mit einer Fettsäure zu verestern, um eine grenzflächenaktive Zusammensetzung zu erhalten.

12. Verfahren nach einem der vorangegangenen Ansprüche, wobei das Verfahren den weiteren Schritt umfasst, das NaAlO₂ zu entfernen.

13. Verfahren nach Anspruch 11, wobei das Entfernen von NaAlO₂ ausgeführt wird, indem die Reaktionsmischung mit einem Ionenaustauscherharz behandelt wird.

## Revendications

1. Procédé de préparation d'une composition comprenant des oligomères et/ou des polymères d'un polyol, lesdits oligomères ou polymères étant liés par des ponts éther, le procédé consistant à faire réagir un matériau polyol de départ avec une quantité de NaAlO₂ qui amène le polyol à former au moins des oligomères dimères avec formation concomitante de H₂O.

2. Procédé selon la revendication 1, **caractérisé en ce que** H₂O formée est éliminée de façon continue.

3. Procédé selon la revendication 1, **caractérisé en ce que** ensuite du polyol est ajouté pendant la réaction.

4. Procédé selon l'une quelconque des revendications précédentes, **caractérisé en ce que** NaAlO₂ est utilisé en une quantité comprise entre 0,1 et 10 % (poids pour poids) calculée par rapport à la quantité de matériau polyol de départ.

5. Procédé selon la revendication 4, **caractérisé en ce que** NaAlO₂ est utilisé en une quantité comprise entre 0,3 et 3,5 %.

6. Procédé selon l'une quelconque des revendications précédentes, **caractérisé en ce que** la température à laquelle le polyol est mis à réagir est comprise entre 200°C et 270°C.

7. Procédé selon l'une quelconque des revendications précédentes, **caractérisé en ce que** la réaction est effectuée à une pression inférieure à 1 bar.

8. Procédé selon l'une quelconque des revendications précédentes, **caractérisé en ce que** la composition résultante est un mélange de substances choisies parmi des monomères, des dimères, des trimères, des tétramères ou des oligomères supérieurs du polyol.

9. Procédé selon l'une quelconque des revendications précédentes, **caractérisé en ce que** le polyol qui est mis à réagir est le glycérol ou un de ses oligomères.

10. Procédé selon la revendication 8, **caractérisé en ce que** la composition résultante est séparée en monomères, dimères, trimères, tétramères ou oligomères supérieurs du polyol.

11. Procédé selon la revendication 1, qui comprend en plus l'étape d'estérification de la composition résultante ou d'une partie de celle-ci par un acide gras pour obtenir une composition tensioactive.

12. Procédé selon l'une quelconque des revendications précédentes, **caractérisé en ce que** le procédé comprend ensuite l'élimination de NaAlO₂.

13. Procédé selon la revendication 11, **caractérisé en ce que** l'élimination de NaAlO₂ s'effectue en traitant le milieu réactionnel par une résine échangeuse d'ions.
